# EUROPEAN PATENT SPECIFICATION

(11) **EP 0 374 929 B1**
(45) Date of publication and mention of the grant of the patent: **25.03.1998**
(21) Application number: 89123685.3
(22) Date of filing: 21.12.1989
(51) Int. Cl.: C07D 257/02, C07D 259/00, C07D 255/02

(54) **Process for preparing mono-N-alkylated polyazamacrocycles**
Verfahren zur Herstellung von mono-N-alkylierten Polyazamacrocyclen
Procédé pour la préparation des polyazamacrocycles mono-N-alkylés

(30) Priority: 22.12.1988 US 289163
(43) Date of publication of application: 27.06.1990
(73) Proprietor: THE DOW CHEMICAL COMPANY, Midland, Michigan 48674 (US)
(72) Inventor: Kruper, William, J. Jr., Sanford Michigan (US)
(74) Representative: Huber, Bernhard, Dipl.-Chem.

(56) References cited:
- EP-A- 0 232 751
- EP-A- 0 287 436
- EP-A- 0 296 522
- JOURNAL OF THE AMERICAN CHEMICAL SOCIETY vol. 110, 1988, pages 6266,6267; M.K. MOI et al.: "The Peptide Way to Macrocyclic Bifunctional Chelating Agents: Synthesis of 2-(p-Nitrobenzyl)-1,4,7,10-tetraazacyclododecane-N,N',N'',N'''-tetraacetic Acid and Study of Its Yttrium(III) Complex"
- HELVETICA CHIMICA ACTA vol.69, fasc. 8, 1986, pages 2081-2086, Basel, CH; M. Studer et al.: "Metal Complexes with Macrocyclic Ligands"
- TOP. CURR. CHEM. vol. 121, 1984, pages 158-179; T.A. KADEN: "Synthesis and Metal Complexes of Aza-Macrocycles with Pendant Arms having Additional Ligating Groups"

## Description

The present invention concerns a process for preparing mono-N-alkylated polyazamacrocycles.

T. A. Kaden, *Top.Curr.Chem.* 121, 157-75 (1984) has shown that simple alkylative techniques using electrophiles and polyazamacrocycles for the production of mono-N-functionalized polyazamacrocyles result in the formation of a mixture of mono, bis, and tris alkylated products which are difficuLt to seperate and purify. To overcome this problem, large excesses of macrocycle, for example 5-10 equivalents relative to electrophile, have been employed by M. Studer et al., *Helv.Chim.Acta.* 69, 2081-86 (1986) and by E. Kimura et al., *J.Chem.Soc.Chem. Commun.* 1158-59 (1986). Additionally, auxiliary bases have used together with large excesses of macrocycle to obtain the mono-N-alkylation product by M. Studer et al., *supra.* Purification of the desired mono-N-alkylated product from the large excess of starting material and inorganic salt is a major problem associated with these methods. Additionally, the cost associated with employing a large excess of expensive reagent is prohibitive.

Other mono-N-alkylation attempts by F. Wagner et al., *Inorg. Chem.* 15, 408 (1976) involve selective deprotonation of a transistion metal complex of a polyazamacrocycle with strong base followed by alkylation with methyl iodide. Further synthetic manipulation of this compound nesscessitates metal removal and purification which can be tedious.

Additional synthetic routes which afford mono-N-functional polyazamacrocycles involve lengthy, protection, functionalization, deprotection schemes which are divergent and not always general. For example, see P. S. Pallavincini et al., *J.Amer.Chem.Soc.* 109, 5139-44 (1987) and published European patent application 0 232 751 (1984).

EP-A-0 287 436 discloses a process for the preparation of monoalkylated tetraazamacrocycles by reacting an about five-fold excess of a tetraazamacrocycle starting compound with an electrophilic organic compound.

In light of these limitations on mono-N-alkylation techniques existing in the art, it would be desirable to employ a direct alkylation approach which would not be reliant upon the use of excess macrocycle and an auxiliary base and yet would be selective for the desired mono-N-alkylated product.

Surprisingly, the present invention provides such a process for preparing selectively the mono-N-alkylated products without the use of large excesses of macrocycle and auxiliary base. The present invention is directed to a novel process for preparing a mono-N-alkylated polyazamacrocycle which comprises reacting an electrophile with between one to two equivalents of a polyazamacrocycle containing (i) at least two secondary amines, which are chemically equivalent, (ii) optionally O and S as other hetero atoms and (iii) a methylene moiety (-CH₂-)ₙ, wherein n is from 2-4, as spacer between the hetero atoms, in a solvent which will not donate a proton to the reaction under the process conditions with the proviso, that the polyazamacrocycle is not a twelve-membered macrocycle. This process results in a greater selectivity for the desired mono-N-alkylated product compared to the bis, tris, tetra or higher N-alkylated products.

The present invention concerns the reaction of an appropriate electrophile (E) with a free base polyazamacrocycle (M) in a relatively non-polar, preferrably relatively aprotic solvent. The ratio of E to M is from one E to two M equivalents, and most preferrably from about one E to about one M equivalent.

The present process is very general and can produce selective mono-N-alkylation products with various polyazamacrocycles and electrophil es. A slight excess of polyazamacrocycle is preferred to insure complete conversion of the electrophile; however, only small differences in isolated yield occur when larger excesses of the polyazamacrocycle are used.

The polyazamacrocycles used in the present invention must contain at least two nitrogen atoms. However, the polyazamacrocycles can also contain other hetero moieties such as oxygen atoms (polyoxazamacrocycles) or sulfur atoms (polythioazamacrocycles). Also included in the term polyazamacrocycles are any symmetrical rings such as polyazamacrocycle, azaoxamacrocycle or bicyclopolyazamacrocycle (i.e. bridged moieties or fused rings, but the mono-N-alkylation reaction occurs on the nitrogen atom of a saturated ring) which bears secondary amines capable of mono-N-alkylation. Preferably the secondary amines are all chemically equivalent. The macrocycle preferably has a plane of symmetry and the total number of hetero atoms (O, S, N) should preferably total an even integer. The final macrocycle also contains a methylene moiety, (-CH₂-)ₙ , where n is from 2-4, as a spacer between the hetero atoms. The term "polyazamacrocycles" means all of these heterocyclic posibilities and includes, for example, 1,4-diazacyclohexane, 1,3,7-triazacyclononane, 1,4,8,11-tetraazacyclotetradecane, 1,4,7,10,13-pentaazacyclopentadecane, 1,4,7,10,13,16-hexaazacyclooctadecane or 1,7,13-triaza-4,10,16-trioxacyclooctadecane, and the like.

The electrophile (RX) used in the present process is one capable of accepting a pair of electrons from one of the nitrogen atoms in the polyazamacrocycle. A covalent bond results from the reaction between the electrophile and polyazamacrocycle and selective mono-N-alkylation results. In the term RX, the R term is a moiety which will yield the desired mono-N-alkylated product, particularly any alkyl, alkylaryl, or alkylheteroaryl functionality. Some examples of the R term are C₁-C₄ alkyl, cyano, pyridinyl, CO₂R¹ or CON(R¹)₂ where R¹ is H, C₁-C₄ alkyl, C₁-C₄ alkaryl where the aryl portion can be a 5 or 6 member ring having carbon, sulfur, nitrogen or oxygen atoms. The X term is any leaving group. Such leaving groups are well known in the art such as, for example, chloro, bromo, iodo, acetate, trifluoroacetate, triflate, mesylate, diazo, brosylate and other similar known groups. Some suitable electrophiles are, for example, *d.l*-2-bromo-4-N-phthalamidobutanoic acid isopropyl ester, *d,l*-2-bromo-4-(4-nitrophenyl)butanoic acid isopropyl ester, *d,l*-2-bromo-4-(4-nitrophenyl)butanoic acid methyl ester, 4-nitrocinnamyl bromide, 4-nitrophenethyl bromide, 4-nitrobenzyl bromide, benzyl bromide and other similar known groups.

Concentration of the reagents is not critical for the present process but for economic reasons and reasonable reaction times, the present process is preferrably conducted under non-dilution conditions such as 1 X 10⁻³M to 2M in each reagent. Mono-N-alkylations conducted under these concentrations afford the desired product in good to excellent yield with unexpectedly high selectivities. The mono-N-alkylated polyazamacrocycle is obtained in a selectivity of at least 40 percent, preferrably from 50 to 98 percent, compared to the bis, tris and/or tetra N-alkylated products.

The solvent used for the present process is one which will not promote a proton transfer. A suitable solvent is a relatively non-polar, relatively aprotic solvent such as, for example, chloroform (CHCl₃), carbon tetrachloride (CCl₄), methylene chloride (CH₂Cl₂), tetrahydrofuran, 1,4-dioxane, and acetonitrile, or a relatively non-polar, relatively aprotic hydrocarbon solvent such as, for example, benzene, cyclohexane, n-hexane, and toluene, or a relatively non-polar, protic alcohol such as, for example, n-butanol, t-butanol, isopropanol, n-hexanol and the like. The preferred solvents are the relatively non-polar, relatively aprotic solvents, especially chloroform. The term "relatively aprotic" means that the solvent does not donate a proton to the reaction under the process conditions. The term "relatively non-polar" means that the solvent has a relatively low dipole moment, as seen from the above listed examples of suitable solvents.

Temperature employed is in the range of from -78 to 100°C, preferrably from -25 to 40°C, more preferrably from 0 to 25°C. The time of the reaction, at a temperature of from 0 to 25°C, is from 10 to 24 hours. The time of the reaction will vary inversely with the temperature; however, the lower temperatures are preferred to aid in selectivity of the desired mono-N-alkylated product.

The absence of base from the displacement conditions of the reaction helps to prevent epimerization of chiral electrophil ic reagents. Hence, optically active mono-N-alkylated adducts are potentially available by this present process. The product formed will possess the inverted optical form from the starting electrophile reagent. For example, the resulting mono-N-alkylated polyazamacrocycle product has the opposite optical configuration from the optically active α-haloacid ester.

The present process does not require the presence of an auxiliary base. Although trace amounts of such bases may be used, the process is conducted in the substiantial absence of an inorganic auxiliary base such as, for example, sodium hydroxide, lithium hydroxide, potassium hydroxide, sodium carbonate, potassium carbonate and the like, or of an organic auxiliary base such as, for example, triethylamine, trimethylamine, pyridine, 4-N,N-diethylaminopyridine, diazobicyclododecane, diazobicyclononane and the like.

Futhermore, the absence of strong base and a low temperature allows for incorporation of other latent functionalities into the periphery of the polyazamacrocycle. For example, both ester and phthalamido functionalities are tolerant of the mild conditions of the present process. A procedure in the art [M. Studer et al., *Helv.Chim.Acta.* 69, 2081-86 (1986)] which employes lithium hydroxide in polar protic solvents such as aqueous ethanol or methanol may be expected to hydrolyze these groups.

The present process has been used to make valuable synthetic precursors to bifunctional chelator molecules for radioactive pharmaceuticals. [See for example, European Patent Application 89 111 497.7 and European published application 296,522, published December 28, 1989, by W. J. Kruper et al.] The brevity of the five step synthetic approach to these compounds, of which the present mono-N-alkylation step is critical, is superior to the nine step process for other pharmaceutical products by M. K. Moi et al., *J.Amer. Chem.Soc.* 110, 6266-27 (1988).

The mono-N-alkylation process of this invention depends upon the availability of the desired electrophile. In the case of substituted α-haloacid esters, commercially available acids may be brominated and converted to an ester in a one pot, two step procedure using N-bromosuccinimide [see D. N. Harpp et al., *J.Org.Chem.* 40, 3420-27 (1975)]. This ionic bromination procedure is superior to the standard Hell-Vollard-Zelinski process and allows for exclusive alpha halogenation of alkanoic acids which contain even reactive benzyl groups. Optically active α-haloacids are readily available from the corresponding amino acids using recent advances in diazotization chemistry [see B. Koppenhoefer et al., "Organic Synthesis", Vol. 66, Ed. C. Heathcock, Pub. Wiley-Interscience, N.Y., pp. 151-57 (1988)]. Other electrophiles used in this invention may be prepared by procedures known in the art or can be purchased commercially.

The polyazamacrocycles and similar cryptand ligands may be prepared by well documented methods which utilize a template effect in a multi-step procedure. For example see T. S. Adkins et al., *J.Amer.Chem.Soc*. 96, 2268-70 (1974) and a review article by T. J. Adkins et al., *Organic Syntheses* 28, 86-97 (1978). With advent of this versatile procedure, a number of these symmetrical macrocycles are now commercially available.

The invention will be further clarified by consideration of the following examples, which are intended to be purely exemplary of the use of this invention.

### General Experimental

In the following examples, the equipment used was as follows:
Mass spectra were obtained on either a Finnigan TSQ mass spectrometer or a VG ZAB-MS high resolution mass spectrometer;
¹H and ¹³C NMR spectra were obtained using a Varian VXR-300 spectrometer; and
IR were recorded on a Nicolet SSX FT/IR instrument.

In the following examples, all solvents were Fisher HPLC grade materials. All preparative chromatraphy of organic compounds was performed using flash chromatography techniques by W. C. Still et al., *J.Org.Chem.* 43, 2923-2925 (1978) and employed the following solvent systems:
Solvent System 1 - CHCl₃:CH₃OH:conc. NH₄OH 2:2:1 (V:V:V);
Solvent System 2 - CHCl₃:CH₃OH:conc. NH₄OH 12:4:1 (V:V:V); and
Solvent System 3 - CHCl₃:CH₃OH:conc. NH₄OH 16:4:1 (V:V:V).

R_{f} values are reported using these solvent systems and commercially available silica plates.

1,4,7,10-Tetraazacyclododecane and 1,4,7,10,13-pentaazacyclopentadecane were purchased from Parrish Chemical Co.

1,4,8,11-Tetraazacyclotetradecane was purchased from Aldrich Chemical Co.

The following terms used in the examples are defined as follows:
conc. means concentrated;
dec means decomposition; and
% means percent.

### Preparation of electrophiles

### Example A

### Preparation of d,l-2-bromo-4-(4-nitrophenyl)butanoic acid methyl ester.

To a solution of 5 mL of carbon tetrachloride and 15 mL of thionyl chloride was added 10.46 g (0.05 mole) of 4-(4-nitrophenyl)butanoic acid under a nitrogen atmosphere. The solution was brought to reflux for one hour with initial rapid liberation of hydrogen chloride and sulfur dioxide. Following the gaseous liberation, 11.0 g (0.06 mole) of N-bromo-succinimide in 25 mL of carbon tetrachloride and 3 drops of 48% aqueous hydrogen bromide catalyst was added to the warm solution whereupon bromine liberation occurred. The dark red solution was refluxed for an additional 15 minutes, cooled and poured into 100 mL of methanol with stirring. TLC (60:40 ethyl acetate:n-hexane) indicated a product characterized by R_{f} = 0.69. The excess solvent was removed and the dark red oil was filtered through a flash silica gel pad 1 in. X 6 in. (2.5 cm X 15.2 cm) using methylene chloride as the eluent. Evaporation of solvent gave 14.5 g of a colorless oil which was an 85:15 mixture of the title product:methyl ester of the starting material and was further characterized by:
¹H NMR (CDCl₃)
   δ8.16(d), 7.38(d), 4.20(dd), 3.79(s), 2.88(m);
¹³C NMR (CDCl₃)
   δ169.6, 147.5, 129.3, 123.7, 53.0, 44.4, 35.5, 33.0.

### Example B

### Preparation of d,l-2-bromo-4-(4-nitrophenyl)butanoic acid isopropyl ester.

The title ester was obtained as a clear oil in 50% yield after quenching the crude acid chloride with isopropanol and chromatographic purification. The product (R_{f} = 0.73, methylene chloride) was devoid of any unbrominated ester and was further characterized by:
¹H NMR (CDCl₃)
   δ8.16(d), 7.38(d), 5.05(septet), 4.14(dd), 2.88(m), 2.39(m), 1.29(d);
¹³C NMR (CDCl₃)
   δ168.7, 147.7, 129.3, 123.8, 69.9, 45.1, 35.6, 33.0, 21.5, 21.2.

### Example C

### Preparation of trans-p-nitrocinnamyl bromide.

In 35 mL of dry acetonitrile under nitrogen atmosphere was added 7.3 g (27.9 mmole) of triphenylphosphine. To the solution was added dropwise over 15 minutes 4.31 g of bromine (27.0 mmole) while cooling the reaction mixture to maintain the temperature between 0-10°C. The solution was allowed to warm to room temperature (about 22-25°C) and 5.0 g (27.9 mmole) of p-nitrocinnamyl alcohol (Pfaultz & Bauer Chemical Co.) was added as a slurry in 50 mL of acetonitrile which caused an exothermic reaction, temperature about 45°C. The resulting dark red solution was heated for one hour at 60°C with stirring and then poured into 500 mL of ether. Triphenylphosphine oxide precipitated from the solution after standing overnight (about 16 hours). The solution was filtered and 15 g of flash silica gel was added to the solution, followed by solvent removal. The resulting powder matrix was applied to a 3 in. X 8 in. (7.6 cm X 20.3 cm) flash column and the desired product was eluted with hexane, followed by 20% ethyl acetate in hexane to yield 5.8 g (23.9 mmole) of the desired pure *trans* product in 86% yield (MP = 75-76°C, R_{f} = 0.81, 60:40 ethyl acetate:n-hexane) and was further characterized by:
¹H NMR (CDCl₃)
   δ8.17(d), 7.51(d), 6.70(dd), 6.56(dt), 4.16(dd);
¹³C NMR (CDCl₃)
   δ147.3, 142.1, 132.0, 129.8, 127.2, 123.9, 31.8.

### Example D

### Preparation of d,l-2-bromo-4-(N-phthalamido)butanoic acid isopropyl ester.

Into a flask equipped with a trap and water condensor was placed 14.8 g (100 mmole) of phthalic anhydride, 10.3 g (100 mmole) of γ-aminobutyric acid (Aldrich Chemical Co.), 1.3 mL of triethylamine and 150 mL of toluene. The mixture was brought to reflux and 1.75 mL of water was removed azeotropically over 1.5 hours. The solution was allowed to cool and stand overnight (about 16 hours). The resulting white crystals that formed were filtered, washed with hexane and dried. The crude crystals were then washed with 250 mL of 5% aqueous hydrogen chloride and 100 mL of cold water. After drying, 19.0 g (81.5 mmole) of 4-(N-phthalamido)butanoic acid was obtained in 82% yield (MP = 114.5-115.5°C, recrystallized from 30% methanol in water) and further characterized by:
¹H NMR (CDCl₃)
   δ7.84(dd), 7.72(dd), 6.05(bs), 3.77(t), 2.42(t), 2.02(p);
¹³C NMR (CDCl₃)
   δ177.9, 169.4, 134.0, 123.3, 37.1, 31.2, 23.6.

The above prepared 4-(N-phthalamido)butanoic acid was reacted by the procedure of Example A and B to obtain the title ester as a white solid, yield 68%, (MP = 72-74.5°C, R_{f} = 0.38, chloroform) after flash silica gel chromatography using chloroform as an eluant, and further characterized by:
¹H NMR (CDCl₃)
   δ7.85(dd), 7.73(dd), 5.04(septet), 4.23(dd), 3.85(dt), 2.51(m), 2.36(m), 1.29(d), 1.26(d).

### Process of the invention.

### Example 1 (not an example of the present invention)

### Preparation of 1,4,7,10-tetraaza-1-[(4-nitrophenyl)methyl]cyclododecane.

The free base, 3.5 g (20.3 mmoles), of 1,4,7,10-tetraazacyclododecane and 1.7 g (7.87 mmoles) of p-nitrobenzyl bromide in 50mL ofchloroform were stirred under nitrogen for 24 hours at 25°C. The chloroform slurry of hydrobromide salt was then applied to a 1 in. X 17 in. (2.5 cm X 43.2 cm) column of flash silica gel (Solvent System 3). The product, 2.13 g (6.93 mmoles) was obtained as a pale yellow solid (MP = 128-29°C, R_{f} = 0.58 Solvent System 3) in 88% yield and further characterized by:
¹H NMR (CDCl₃)
   δ8.18(d), 7.49(d), 3.69(s), 2.82(t), 2.70(t), 2.59(m);
¹³C NMR (CDCl₃)
   δ147.2, 128.4, 123.8, 58.8, 51.7, 47.1, 46.3, 45.1.

### Example 2 (not an example of the present invention)

### Preparation of 1,4,7,10-tetraaza-1-[2-(4-nitrophenyl)ethyl]cyclododecane.

To a stirred solution of 3.5 g (20.3 mmole) of 1,4,7,10-tetraazacyclododecane in 50 mL of pentene stabilized chloroform was added dropwise over five minutes with vigorous stirring under a nitrogen atmosphere 4.0 g (17.4 mmoles) of 1-bromo-2-(4-nitrophenyl)ethane (Aldrich Chemical Co.). The stirring was continued overnight at about 25°C whereupon crystals of amine hydrobromide precipitated from solution. The contents of the flask were applied to a flash silica gel column 1 in. X 18 in. (2.5 cm X 45.7 cm) which had been pre-eluted with 5% methanol in chloroform and 200 mL of this solution was applied as an eluent, followed by elution with Solvent System 3. The p-nitrostyrene (1.45 g, 9.7 mmole, R_{f} = 0.98, Solvent System 2) was cleanly separated from 2.27 g (7.06 mmole) of the desired product as an orange yellow oil (yield 40.6%) which solidified upon standing (R_{f} = 0.73, Solvent System 2). A sample was recrystallized [MP = 146.5-148.5°C (dec)] from chloroform/cyclohexane and was further characterized by:
¹H NMR (CDCl₃)
   δ8.14(d), 7.40(d), 2.91(t), 2.77(t), 2.72(t), 2.50(t), 2.60(s);
¹³C NMR (CDCl₃)
   δ148.5, 129.6, 123.4, 55.5, 51.4, 46.9, 45.9, 45.1, 33.7.

### Example 3 (not an example of the present invention)

### Preparation of 1,4,7,10-tetraaza-1-[1-carbomethoxy-3-(4-nitrophenyl)propyl]cyclododecane.

To a stirred solution of 1.72 g (10.0 mmole) of 1,4,7,10-tetraazacyclododecane in 17 mL of pentene stabilized chloroform was added 2.07 g (5.82 mmole) of crude *d,l*-2-bromo-4-(4-nitrophenyl)butanoic acid methyl ester (prepared by the procedure of Example A) over a five minute peroid under a nitrogen atmosphere. The reaction mixture was stirred for 48 hours at about 25°C and TLC (Solvent System 2) indicated the conversion to the desired monoalkylation product (R_{f} = 0.73, ninhydrin, iodine, and UV active). The yellow chloroform solution was applied to a 1 in. X 16 in. (2.5 cm X 40.6 cm) flash silica gel column which had been preeluted with 5% methanolic chloroform, followed by elution with 250 mL of the preelution solvent system, and then elution with Solvent System 2. The fractions containing the desired product were combined and evaporated affording 2.15 g (5.46 mmole) of the desired product (MP = 156-159°C, recrystallized from chloroform, ether) in 94% yield and further characterized by:
¹H NMR (CDCl₃)
   δ8.14(d), 7.39(d), 3.71(s), 3.39(dd), 2.5-3.0(m), 2.08(m), 2.01(m);
¹³C NMR (CDCl₃)
   δ172.7, 149.3, 146.4, 129.2, 123.6, 62.3, 51.2, 48.9, 47.2, 45.8, 45.4, 32.8, 30.9.

### Example 4

### Preparation of 1,4,7,10,13-pentaaza-1-[1-carboisopropoxy-3-(4-nitrophenyl)propyl]cyclopentadecane.

The purified desired product was obtained as a light yellow oil in 77% yield after chromatography on silica gel (R_{f} = 0.82, Solvent System 2) and further characterized by:
¹H NMR (CDCl₃)
   δ8.14(d), 7.49(d), 5.09(septet), 4.86(s), 3.38(dd,s), 2.5-3.0(m), 2.30(m), 2.11(m), 1.29(d);
¹³C NMR (CDCl₃)
   δ171.9, 149.1, 146.5, 129.5, 123.6, 68.7, 61.9, 49.5, 46.8, 46.7, 45.5, 45.3, 32.9, 32.1, 22.1, 22.0;
IR (CDCl₃) cm⁻¹
   3450, 2860, 1735, 1350, 915.

### Example 5 (not an example of the present invention)

Yields of mono-N-alkylation of p-nitrobenzyl bromide (Aldrich Chemical Co.) with 1,4,7,10-tetraazacyclododecane.

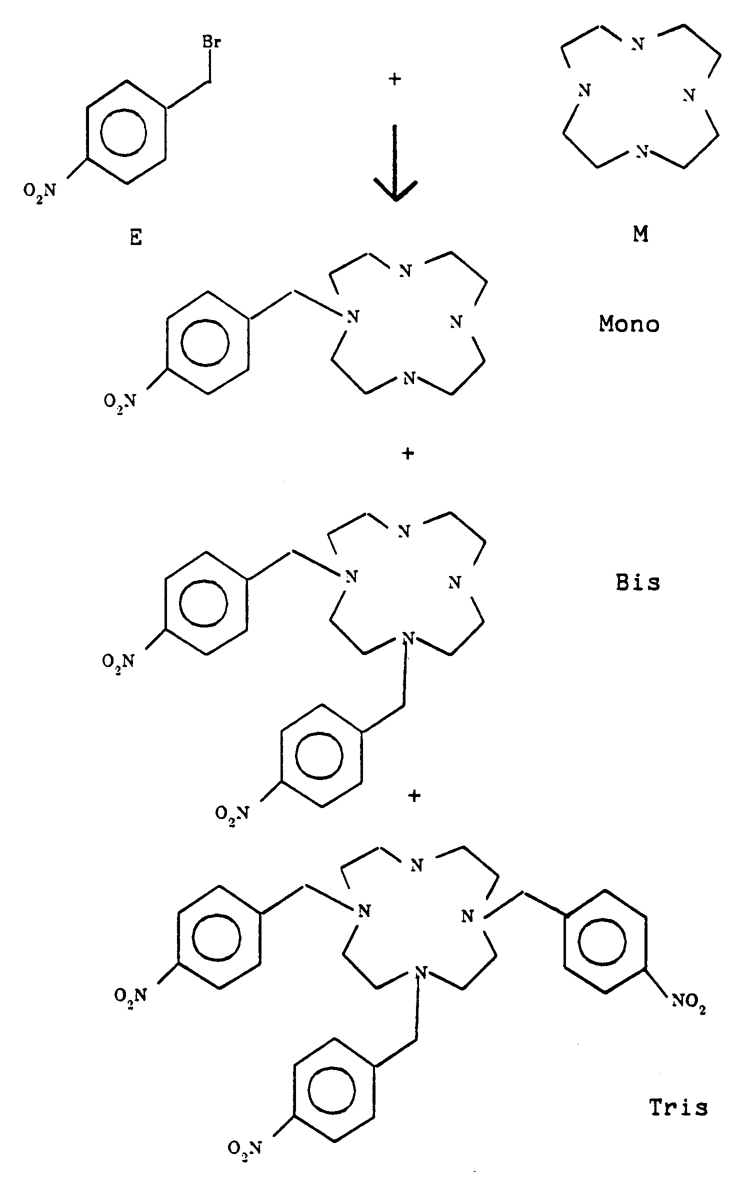

**TABLE I**

| Ex. No. | M:E | [E] MOLARITY | SOLVENT | % ISOLATED YIELD ** | | | MONO:BIS |
|---|---|---|---|---|---|---|---|
| | | | | MONO | BIS | TRIS | |
| 5A | 1:1.0 | 1 | CHCl₃ | 49 | 11 | 5 | 4.5:1 |
| 5B | 1:1.5 | 1 | CHCl₃ | 66 | 10 | 3 | 6.6:1 |
| 5C | 1:1.5 | 4 x 10⁻² | CHCl₃ | 73 | 9 | 2 | 7.4:1 |
| 5D | 1:1.5 | 1 | CH₃OH | 39 | 16 | 3 | 2.4:1 |
| 5E | 1:1.5 | 1 | CH₃(CH₂)₃OH | 43 | 15 | 3 | 3.0:1 |
| A* | 1:1.5 | 1 | CH₃OH*** | 34 | 19 | 7 | 1.8:1 |

| | | | | | | | |
|---|---|---|---|---|---|---|---|
| *A is not a process of the invention. | | | | | | | |
| **Isolated yield of product after flash chromatography; process at 25°C. | | | | | | | |
| ***1.0 equivalents of KCO₃ added relative to bromide. *** | | | | | | | |

### Example 6

### Preparation of various mono-N-alkylpolyazamacrocycles using the process of the invention.

In Table II following, various electrophiles were reacted with polyazamacrocycles which gave yields greated than 40% selectivity for the desired mono-N-alkylpolyazamacrocycle, expressed as percent (%) isolated yield. The process was run using the equivalents and solvents given in Table II and by the procedure of Examples 1-4. Table III following indicates the structure of the desired mono-N-alkylpolyazamacrocycle formed by the reaction in Table II.

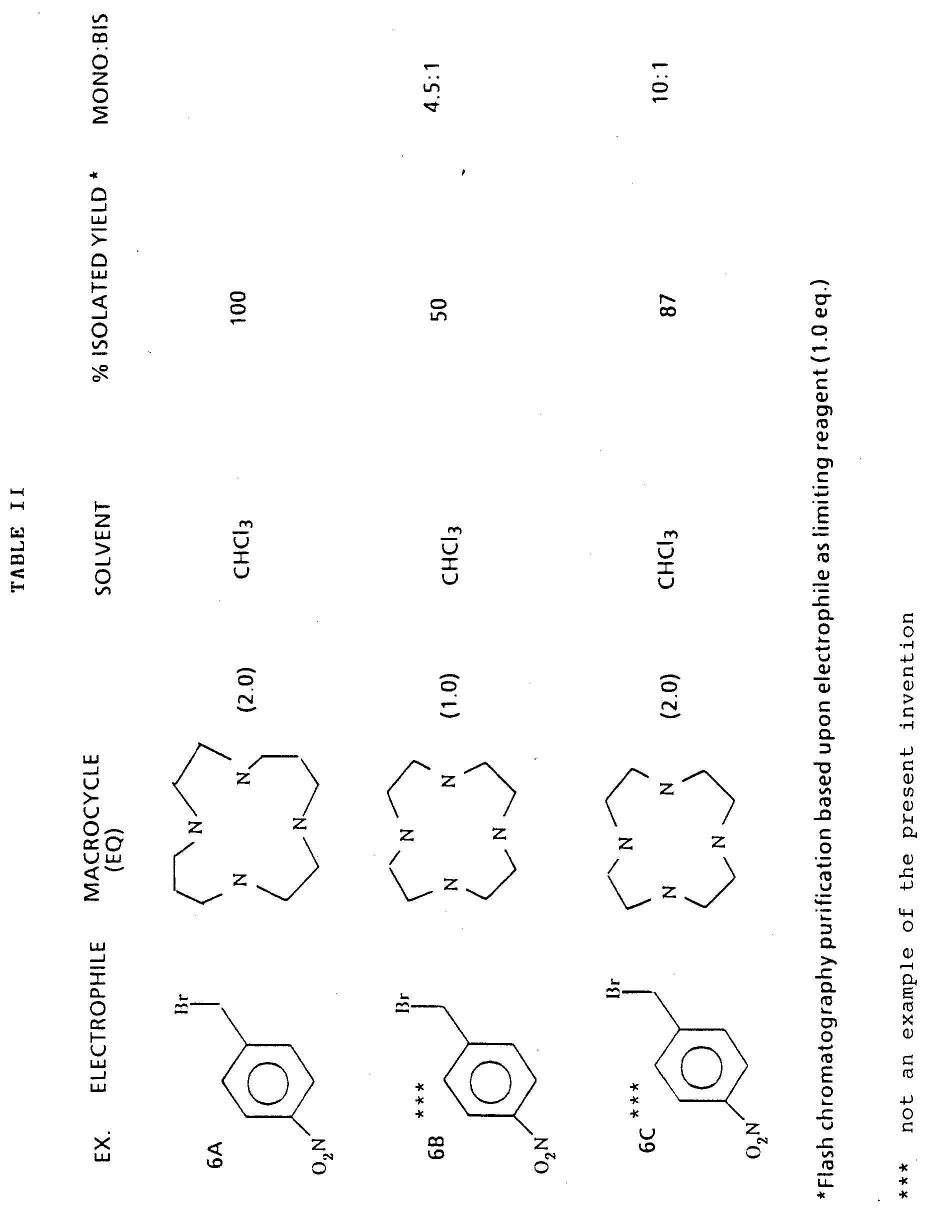

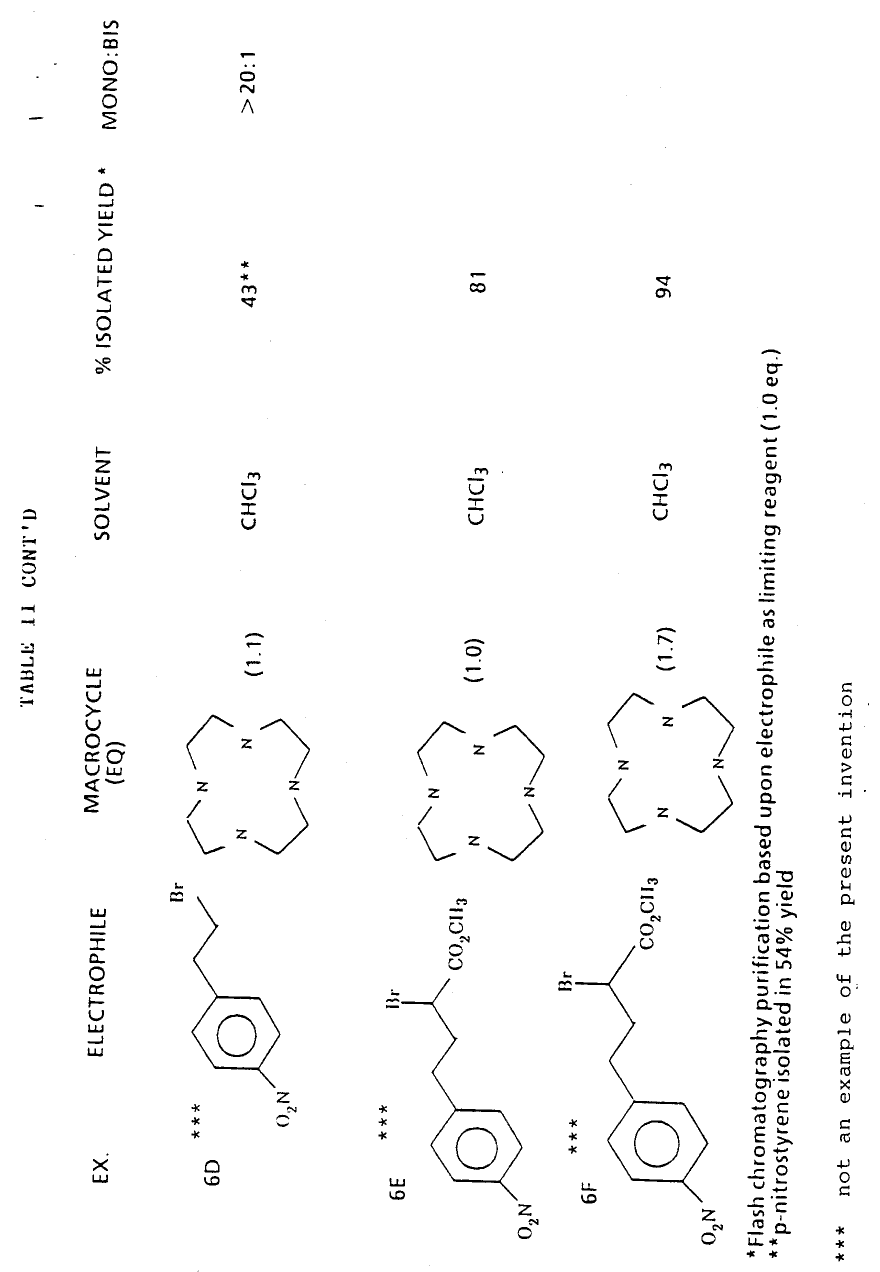

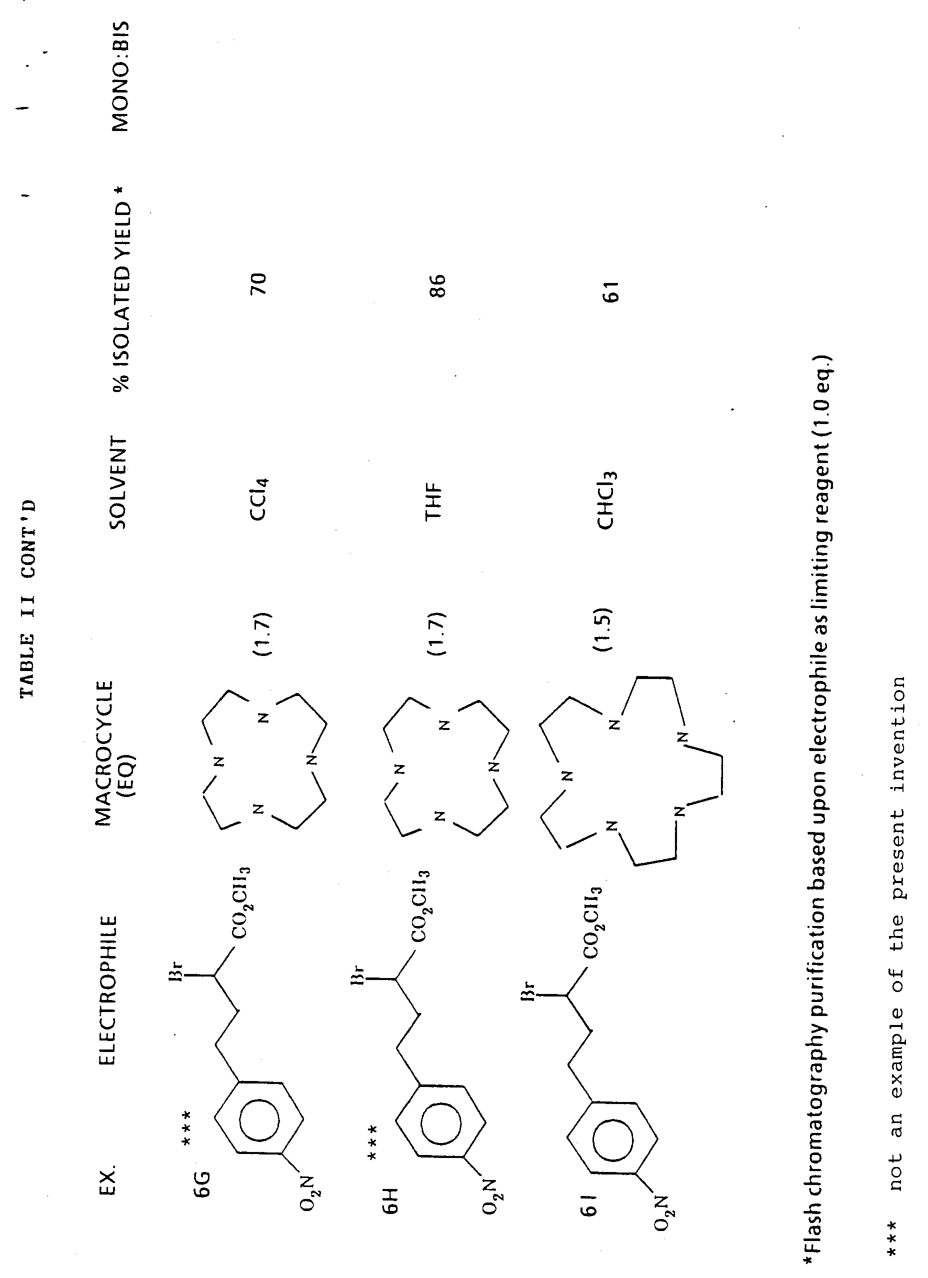

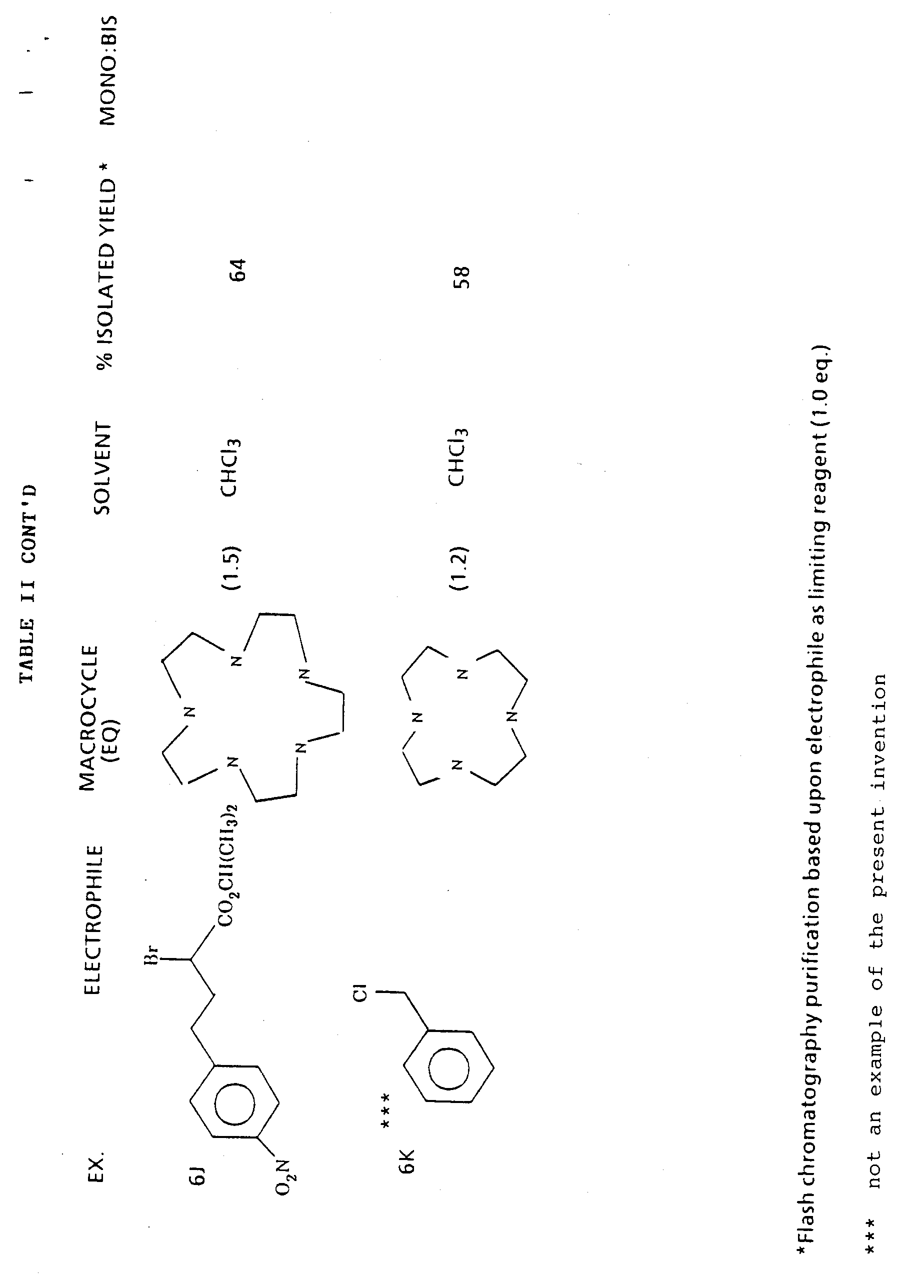

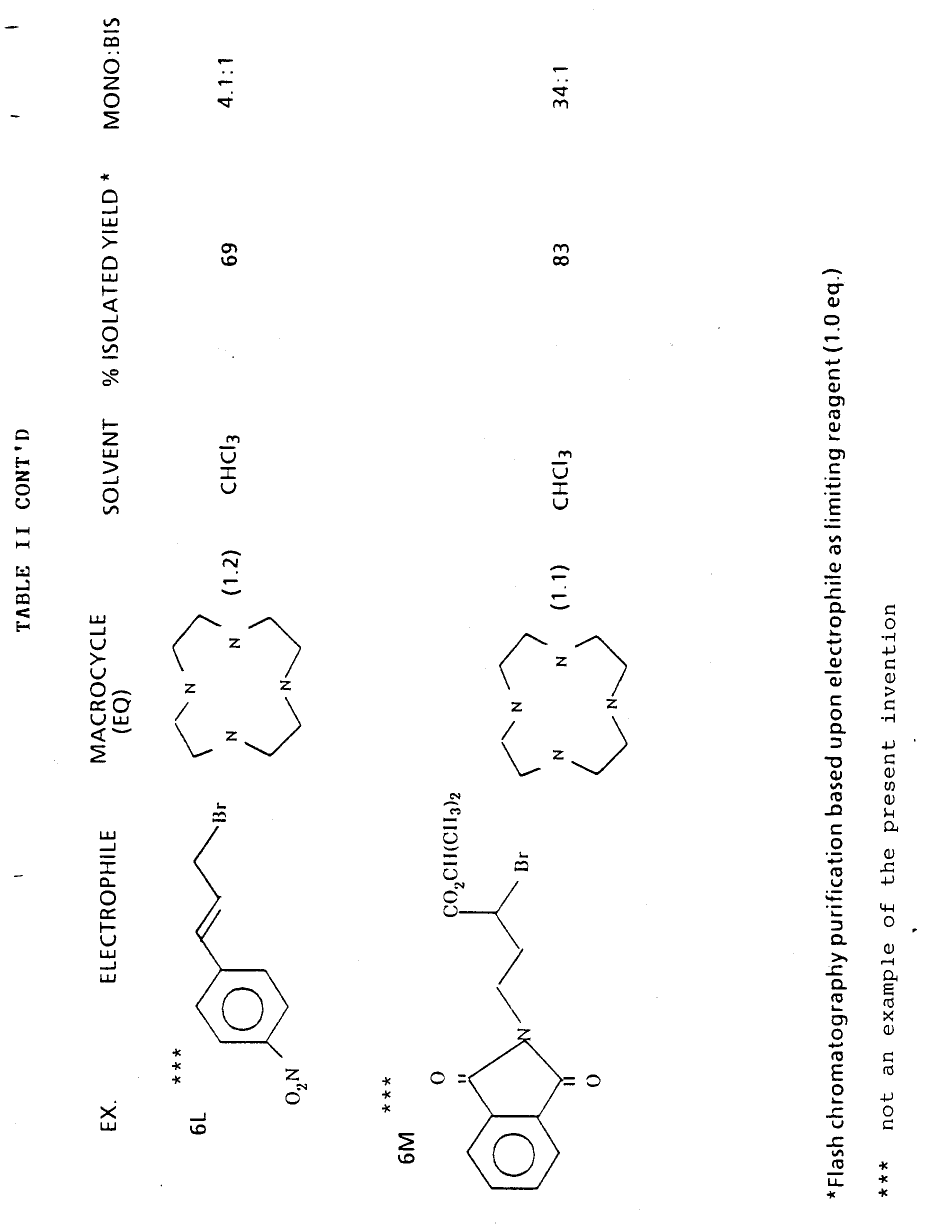

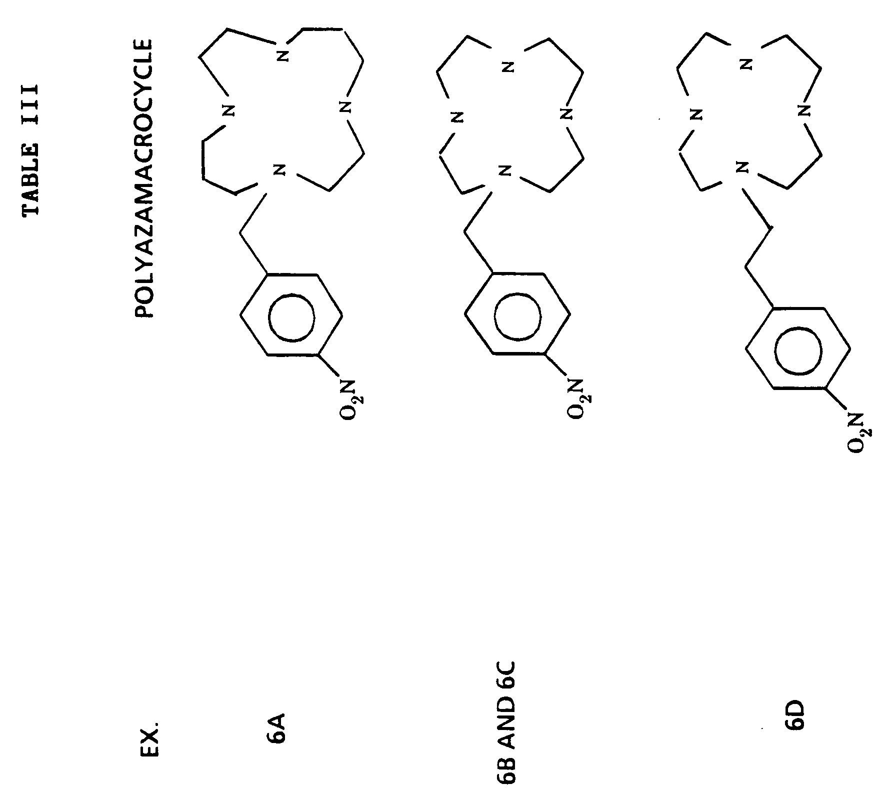

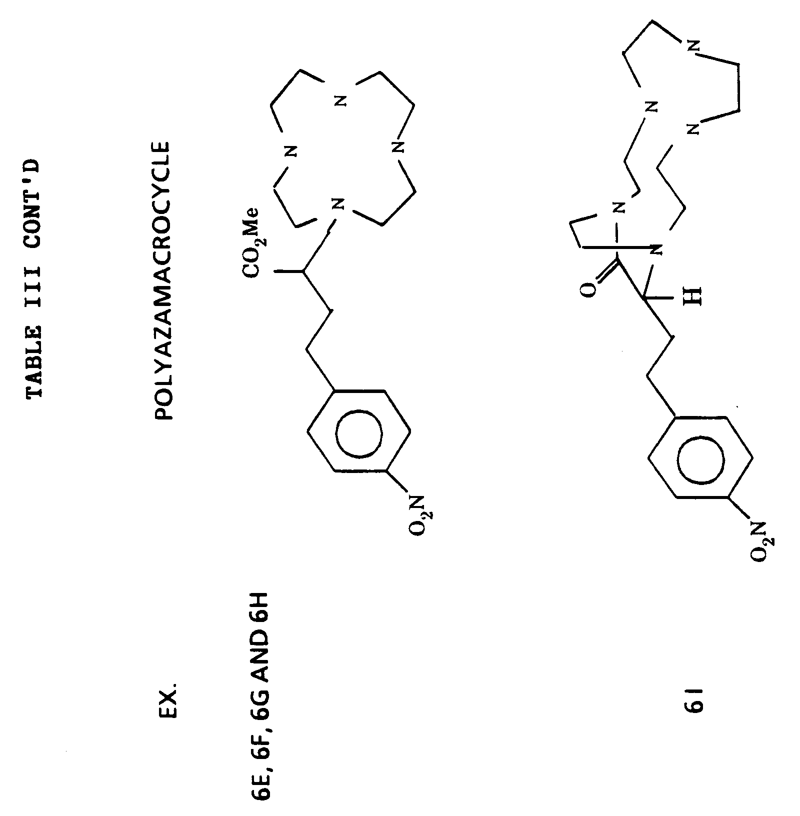

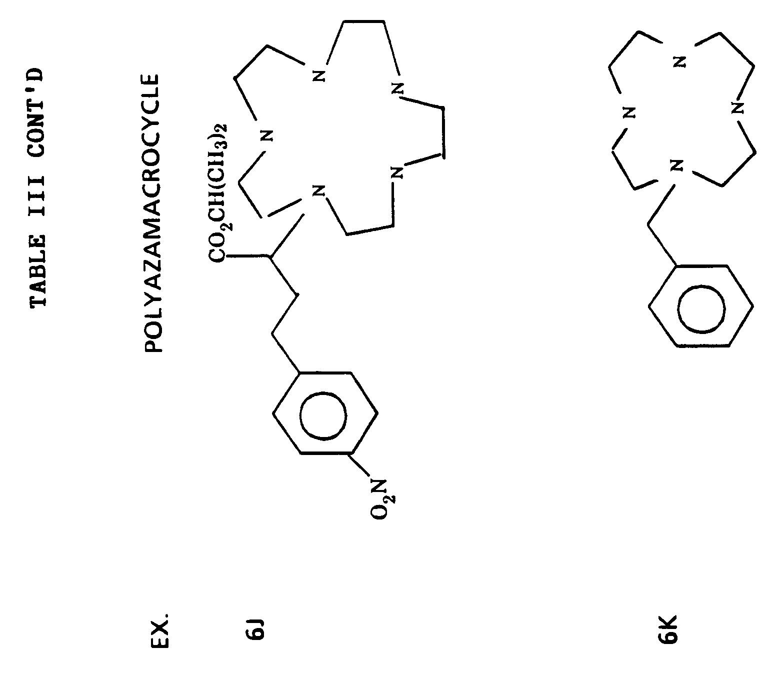

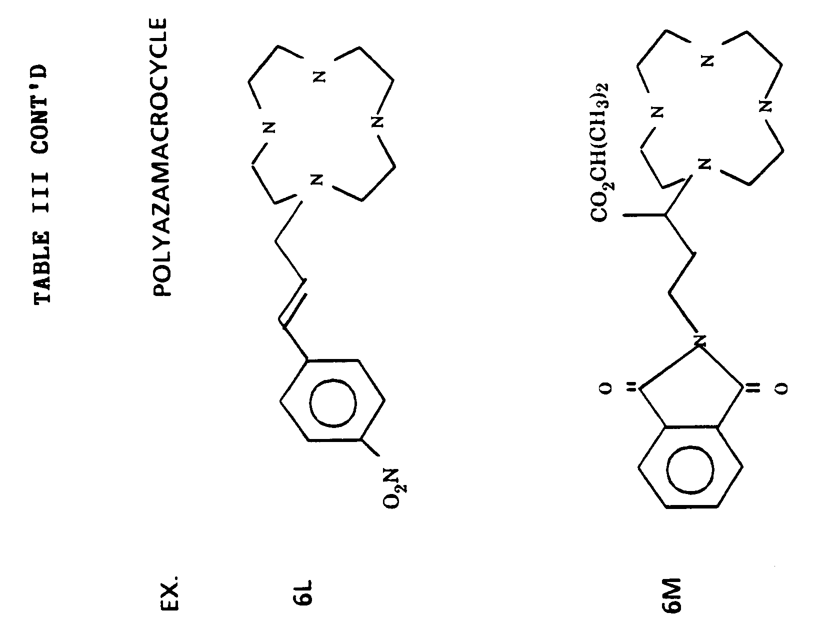

Other embodiments of the invention will be apparent to those skilled in the art from a consideration of this specification or practice of the invention disclosed herein. It is intended that the specification and examples be considered as exemplary only, with the true scope and spirit of the invention being indicated by the following claims:

## Claims

1. A process for preparing a mono-N-alkylated polyazamacrocycle which comprises reacting an electrophile with between one to two equivalents of a polyazamacrocycle containing (i) at least two secondary amines, which are chemically equivalent, (ii) optionally O and S as other hetero atoms and (iii) a methylene moiety (-CH₂-)ₙ, wherein n is from 2-4, as spacer between the hetero atoms, in a solvent which will not donate a proton to the reaction under the process conditions with the proviso, that the polyazamacrocycle is not a twelve-membered macrocycle.

2. The process of claim 1 wherein the solvent is a non-polar, aprotic solvent.

3. The process of claim 2 wherein the solvent is chloroform, carbon tetrachloride, methylene chloride, tetrahydrofuran, 1,4-dioxane or acetonitrile.

4. The process of claim 2 or 3 wherein the solvent is chloroform.

5. The process of claim 2 wherein the solvent is a hydrocarbon solvent.

6. The process of claim 5 wherein the solvent is benzene, cyclohexane, n-hexane or toluene.

7. The process of claim 1 wherein the solvent is a non-polar, protic alcohol.

8. The process of claim 7 wherein the solvent is n-butanol, t-butanol, isopropanol or n-hexanol.

9. The process of claim 1 wherein the electrophile is optically active.

10. The process of claim 9 wherein the resulting mono-N-alkylated polyazamacrocycle product has the opposite optical configuration from the optically active electrophile.

11. The process of claim 1 wherein the temperature is from -78 to 100°C.

12. The process of claim 11 wherein the temperature is from -25 to 40°C.

13. The process of claim 12 wherein the temperature is from 0 to 25°C.

14. The process of claim 1 wherein one equivalent of polyazamacrocycle is used relative to the electrophile.

15. The process of claim 1 wherein the mono-N-alkylated polyazamacrocycle is obtained in a selectivity of at least 40 percent.

16. The process of claim 1 wherein the mono-N-alkylated polyazamacrocycle is obtained in a selectivity of from 50 to 98 percent.

## Patentansprüche

1. Verfahren zur Herstellung eines mono-N-alkylierten Polyazamacrocyclus, welches umfaßt Umsetzen eines Elektrophils mit zwischen einem und zwei Äquivalenten eines Polyazamacrocyclus enthaltend (i) mindestens zwei sekundäre Amine, die chemisch äquivalent sind, (ii) gegebenenfalls O und S als weitere Heteroatome und (iii) eine Methyleneinheit (-CH₂-)ₙ, worin n von 2 bis 4 ist, als Zwischenstück zwischen den Heteroatomen, in einem Lösungsmittel, das an die Reaktion unter den Verfahrensbedingungen kein Proton abgibt, unter der Voraussetzung, daß der Polyazamacrocyclus kein 12-gliedriger Macrocyclus ist.

2. Verfahren nach Anspruch 1, worin das Lösungsmittel ein nichtpolares, aprotisches Lösungsmittel ist.

3. Verfahren nach Anspruch 2, worin das Lösungsmittel Chloroform, Tetrachlorkohlenstoff, Methylenchlorid, Tetrahydrofuran, 1,4-Dioxan oder Acetonitril ist.

4. Verfahren nach Anspruch 2 oder 3, worin das Lösungsmittel Chloroform ist.

5. Verfahren nach Anspruch 2, worin das Lösungsmittel ein Kohlenwasserstoff-Lösungsmittel ist.

6. Verfahren nach Anspruch 5, worin das Lösungsmittel Benzol, Cyclohexan, n-Hexan oder Toluol ist.

7. Verfahren nach Anspruch 1, worin das Lösungsmittel ein nichtpolarer, protischer Alkohol ist.

8. Verfahren nach Anspruch 7, worin das Lösungsmittel n-Butanol, t-Butanol, Isopropanol oder n-Hexanol ist.

9. Verfahren nach Anspruch 1, Worin das Elektrophil optisch aktiv ist.

10. Verfahren nach Anspruch 9, worin das resultierende mono-N-alkylierte Polyazamacrocyclus-Produkt die entgegengesetzte optische Konfiguration des optisch aktiven Elektrophils aufweist.

11. Verfahren nach Anspruch 1, worin die Temperatur von -78 bis 100°C beträgt.

12. Verfahren nach Anspruch 11, worin die Temperatur von -25 bis 40°C beträgt.

13. Verfahren nach Anspruch 12, worin die Temperatur von 0 bis 25°C beträgt.

14. Verfahren nach Anspruch 1, worin ein Äquivalent eines Polyazamacrocyclus bezogen auf das Elektrophil verwendet wird.

15. Verfahren nach Anspruch 1, worin der mono-N-alkylierte Polyazamacrocyclus mit einer Selektivität von mindestens 40 % erhalten wird.

16. Verfahren nach Anspruch 1, worin der mono-N-alkylierte Polyazamacrocyclus mit einer Selektivität von 50 bis 98 % erhalten wird.

## Revendications

1. Procédé de préparation d'un polyazamacrocycle mono-N-alkylé, qui comporte le fait de faire réagir un agent électrophile avec de 1 à 2 équivalents d'un polyazamacrocycle comportant :
a) au moins deux fonctions amine secondaire, chimiquement équivalentes,
b) éventuellement, d'autres hétéroatomes qui sont des atomes d'oxygène ou de soufre, et
c) un fragment constitué de groupes méthylène, ―(CH₂)ₙ― où n vaut de 2 à 4, comme groupe espaçant placé entre les hétéroatomes, dans un solvant qui ne fournit pas de protons pour la réaction dans les conditions opératoires,
sous réserve que le polyazamacrocycle ne soit pas un macrocycle à 12 chaînons.

2. Procédé conforme à la revendication 1, dans lequel le solvant est un solvant non-polaire et aprotique.

3. Procédé conforme à la revendication 2, dans lequel le solvant est du chloroforme, du tétrachlorure de carbone, du chlorure de méthylène, du tétrahydrofurane, du 1,4-dioxane ou de l'acétonitrile.

4. Procédé conforme à la revendication 2 ou 3, dans lequel le solvant est du chloroforme.

5. Procédé conforme à la revendication 2, dans lequel le solvant est un hydrocarbure.

6. Procédé conforme à la revendication 5, dans lequel le solvant est du benzène, du cyclohexane, du n-hexane ou du toluène.

7. Procédé conforme à la revendication 1, dans lequel le solvant est un alcool non-polaire et protique.

8. Procédé conforme à la revendication 7, dans lequel le solvant est du n-butanol, du t-butanol, de l'isopropanol ou du n-hexanol.

9. Procédé conforme à la revendication 1, dans lequel l'agent électrophile est optiquement actif.

10. Procédé conforme à la revendication 9, dans lequel le produit, c'est-à-dire le polyazamacrocycle mono-N-alkylé résultant, présente la configuration optique inverse de celle de l'agent électrophile optiquement actif.

11. Procédé conforme à la revendication 1, au cours duquel la température vaut de -78 à 100 °C.

12. Procédé conforme à la revendication 11, au cours duquel la température vaut de -25 à 40 °C.

13. Procédé conforme à la revendication 12, au cours duquel la température vaut de 0 à 25 °C.

14. Procédé conforme à la revendication 1, au cours duquel on utilise 1 équivalent de polyazamacrocycle, par rapport à l'agent électrophile.

15. Procédé conforme à la revendication 1, grâce auquel on obtient un polyazamacrocycle mono-N-alkylé avec une sélectivité d'au moins 40 %.

16. Procédé conforme à la revendication 1, grâce auquel on obtient un polyazamacrocycle mono-N-alkylé avec une sélectivité de 50 à 98 %.
